# EUROPÄISCHE PATENTSCHRIFT

(11) **EP 3 858 893 B1**
(45) Veröffentlichungstag und Bekanntmachung des Hinweises auf die Patenterteilung: **01.07.2026**
(21) Anmeldenummer: 21152712.2
(22) Anmeldetag: 21.01.2021
(51) Int. Cl.: C08G 77/06, C08G 77/46

(54) **VERFAHREN ZUR HERSTELLUNG HOCHREINER HYDROSILYLIERUNGSPRODUKTE**
METHOD FOR THE PREPARATION OF HIGH PURITY HYDROSILATION PRODUCTS
PROCÉDÉ DE FABRICATION DE PRODUITS D'HYDROSILYLATION DE HAUTE PURETÉ

(30) Priorität: 30.01.2020 EP 20154483
(43) Veröffentlichungstag der Anmeldung: 04.08.2021
(73) Patentinhaber: Evonik Operations GmbH, 45128 Essen (DE)
(72) Erfinder: Lobert, Matthias, 45309 Essen (DE); Reibold, Thomas, 45701 Herten (DE)
(74) Vertreter: Evonik Patent Association

(56) Entgegenhaltungen:
- EP-A1- 3 663 371
- DE-T2- 69 310 619
- US-B2- 8 497 338
- MACIEJEWSKI H ET AL: "Silicone waxes-synthesis via hydrosilylation in homo- and heterogeneous systems", JOURNAL OF MOLECULAR CATALYSIS A: CHEMICAL, ELSEVIER, AMSTERDAM, NL, vol. 257, no. 1-2, 1 September 2006 (2006-09-01), pages 141 - 148, XP028015254, ISSN: 1381-1169, [retrieved on 20060901], DOI: 10.1016/J.MOLCATA.2006.04.039
- DATABASE WPI Week 200377, Derwent World Patents Index; AN 2003-817061, XP002799656

## Beschreibung

Die vorliegende Erfindung liegt auf dem Gebiet der Silikone. Sie betrifft insbesondere ein Verfahren zur Herstellung hochreiner Hydrosilylierungsprodukte. Produkte, die nach diesem Verfahren hergestellt werden können und deren Verwendung, insbesondere als grenzflächenaktive Substanzen, werden ebenfalls offenbart.

Unter Hydrosilylierungsprodukten versteht der Fachmann vorzugsweise SiC-verknüpfte, organomodifizierte Siloxane, insbesondere Polyethersiloxane, die mit ihrem weit einstellbaren Tensidverhalten eine industriell sehr wichtige Stoffklasse darstellen.

Hydrosilylierungsreaktionen von SiH-Gruppen tragenden Siloxanen und/oder Silanen mit Verbindungen, die eine C=C-Doppelbindung enthalten, werden kontinuierlich oder diskontinuierlich und in jedem Fall in Gegenwart eines Edelmetall-Katalysators durchgeführt.

Dem Fachmann sind entsprechend katalytisch aktive Verbindungen des Platins, Palladiums, Rhodiums, Rutheniums, Iridiums und Osmiums bekannt.

Im Stand der Technik wird meist auf die Platin metall-katalysierte Anlagerung SiH-Gruppen tragender Siloxane und Silane an olefinisch funktionalisierte Verbindungen, wie z.B. an Allylpolyether zurückgegriffen, wie z.B. in dem Buch "Chemie und Technologie der Silicone", Verlag Chemie, 1960, Seite 43, und in der Patentliteratur beschrieben.

Als Katalysatoren haben sich in der heutigen betrieblichen Praxis überwiegend Pt-Katalysatoren wie Hexachloroplatinsäure (US 2823218), cis-Diammino-platin(II)chlorid und Karstedt-Katalysator (US 3775452) durchgesetzt.

Die Platin-Katalysatoren liegen meist als homogene Katalysatoren im Reaktionsgemisch vor und können vielfach im Endprodukt verbleiben. Durch gestiegene Marktanforderungen besteht allerdings ein immer größerer Bedarf auch an Pt-freien Produkten. Neben ökologischen Aspekten, wie dem Rückgewinn wichtiger Edelmetall-Ressourcen, steht verstärkt auch die Verbesserung der eigentlichen Produktqualität im Fokus.

Hydrosilylierungsprodukte mit erhöhtem Edelmetallgehalt im allgemeinen und erhöhten Pt-Gehalten im Besonderen zeigen meist direkt nach der Herstellung das Problem dunklerer Farbe des Produktes, manchmal verändert sich die Farbe jedoch auch zeitversetzt, beides stellt jedoch einen eindeutigen Qualitätsmangel dar. Ein weiterer Qualitätsmangel ist zudem die mit der Alterung häufig auftretende langsame Bildung von schwarzen Partikeln durch ausfallendes Edelmetall.

Dieser Sachverhalt führt häufig zu Kundenreklamationen und dieser Qualitätsmangel sollte demzufolge behoben werden.

Im Stand der Technik sind diverse Lösungsansätze bekannt, wie dem wissenschaftlichen Artikel Ind. Eng. Chem. Res 2012, 51, 16457-16466 und der darin zitierten Literatur zu entnehmen ist.

Neben Membrantechnologie, Lösemittelextraktion und gezielter Ausfällung ist der Einsatz von Adsorbentien breit etabliert.

Als Adsorbentien eignen sich beispielsweise saure oder basische Ionenaustauscher, Chelatisierungsagenzien oder funktionalisierte Silicagele (WO 2017213809 A1), aktivierte Kohlenstoff (CN 20150692397) oder Carbon black (CN201510199280).

Der Einsatz derartiger Adsorbentien erfordert allerdings einen weiteren zeitraubenden Prozessschritt von 4 Stunden nach der eigentlichen Hydrosilylierungsreaktion wie beispielsweise in WO 2017213809 A1 beschrieben.

Eine effizientere Edelmetallentfernung lässt sich durch den Einsatz geträgerter Katalysatoren erzielen. Die Isolation des Katalysators durch einfache Filtration ist leicht möglich, nachteilig ist hier jedoch, dass der geträgerte Katalysator extra hergestellt werden muss.

Ein auf diesem Konzept beruhendes Verfahren beschreibt US 8497338 B2, wo der Hydrosilylierungsprozess dergestalt durchgeführt wird, dass das Reaktionsmedium durch ein Katalysatorfestbett geführt wird.

Neben dem Problem, dass ein derartiges Festbett ausbluten kann und nach einer gewissen Standzeit mit hohem technischem und personellem Aufwand gewechselt werden muss, sind auch hohe Investitionen nötig, um neue Anlagen zu bauen, die eine Hydrosilylierungsreaktion in kontinuierlicher Fahrweise am Festbett ermöglichen.

DE 693 10 619 T2 offenbart ein Verfahren zur Herstellung von organisch modifizierten Polysiloxanen durch Hydrosilylierung in Anwesenheit eines Platinkatalysators, wobei dieser auf einem festen Träger angeordnet und der feste Träger Aktivkohle ist.

MACIEJEWSKI H ET AL, "Silicone waxes-synthesis via hydrosilylation in homo- and heterogeneous systems", JOURNAL OF MOLECULAR CATALYSIS A: CHEMICAL, ELSEVIER, AMSTERDAM, NL, Bd. 257, Nr. 1-2, doi:10.1016/J.MOLCATA.2006.04.039, ISSN 1381-1169, Seiten 141 - 148, (20060901), XP028015254, offenbart ein Verfahren zur Herstellung von organisch modifizierten Polysiloxanen durch Hydrosilylierung in Anwesenheit eines Edelmetallkatalysators, wobei Adsorbentien (Aktivkohleteilchen oder vernetzte Styrolpolymere) zum Einsatz gelangen.

In DATABASE WPI, Derwent World Patents Index, vol. 2003, no. 77, Database accession no. 2003-817061, XP002799656 & JP 2003 082103 A 20030319 (GE TOSHIBA SILICONES CO LTD) wird ein Verfahren zur Herstellung von organisch modifizierten Polysiloxanen durch Hydrosilylierung in Anwesenheit eines Edelmetallkatalysators, Platin auf Alumina, offenbart. Es kommen Adsorbentien zum Einsatz, nämlich Alumina oder Zeolithe.

Da Hydrosilylierungsreaktionen überwiegend in einer Batch- oder Semibatch-Fahrweise durchgeführt werden, bestand der Bedarf ein einfaches und besonders preiswertes Verfahren zur Herstellung von Hydrosilylierungsprodukten zu ermöglichen, wobei insbesondere die Bereitstellung besonders reiner Hydrosilylierungsprodukte ermöglicht wird.

Überraschenderweise wurde im Rahmen der vorliegenden Erfindung gefunden, dass die Durchführung einer Edelmetall-katalysierten Hydrosilylierung eines H-funktionellen Siloxans mit einer ungesättigten organischen Verbindung in Gegenwart von Adsorbentien, die als eine weitere, separate Komponente zugegeben werden, zu farbreduzierten und vorzugsweise farblosen, und damit insbesondere zu hochreinen Hydrosilylierungsprodukten führt. Die Farbreduzierung ergibt sich im Vergleich zu einer ansonsten analogen Vorgehensweise allerdings ohne Einsatz von separat zugegebenen Adsorbentien.

Überraschenderweise wurde im Rahmen der vorliegenden Erfindung weiterhin gefunden, dass die Durchführung einer Edelmetall-katalysierten Hydrosilylierung eines H-funktionellen Siloxans mit einer ungesättigten organischen Verbindung in Gegenwart von Adsorbentien, die als eine weitere, separate Komponente zugegeben werden, und Wasser zu qualitativ noch besseren, und noch reineren Hydrosilylierungsprodukten führt.

Gegenstand der vorliegenden Erfindung ist demnach ein Verfahren zur Herstellung von organisch modifizierten Polysiloxanen und/oder Silanen durch Hydrosilylierung, umfassend folgende Schritte:
a) Umsetzung eines SiH-funktionellen Siloxans und/oder Silans mit einer ungesättigten organischen Verbindung in Anwesenheit eines Edelmetallkatalysators sowie optional in Anwesenheit von Wasser,
b) optionale Destillation,
c) abschließende Feststoffabtrennung, insbesondere durch Filtration,
wobei in dem Schritt a) Adsorbentien zum Einsatz gelangen, die als eine weitere, separate Komponente zugegeben werden.

In dem erfindungsgemäßen Verfahren werden demnach zumindest 4 unterschiedliche Komponenten zugegeben: (i) SiH-funktionelles Siloxan und/oder Silan; (ii) ungesättigte organische Verbindung; (iii) Edelmetallkatalysator; (iv) Adsorbentien. Die zuzugebenenden Komponenten (iii) und (iv) sind unterschiedliche Komponenten.

Ebenfalls offenbart werden Hydrosilylierungsprodukte, hergestellt nach dem erfindungsgemäßen Verfahren, sowie deren Verwendung, z.B. als grenzflächenaktive Substanzen.

Die Begriffe "Polysiloxan" und "Siloxan" werden im Sinn dieser Erfindung synonym verwendet.

Die erfindungsgemäßen Gegenstände werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Werden in der vorliegenden Erfindung chemische (Summen-)Formeln verwendet, so können die angegebenen Indizes sowohl absolute Zahlen als auch Mittelwerte darstellen. Bei polymeren Verbindungen stellen die Indizes vorzugsweise Mittelwerte dar. Bei Prozentangaben handelt es sich, wenn nicht anders angegeben, um Angaben in Gewichtsprozent. Sind nachfolgend Messwerte angegeben, so sind diese Messungen, wenn nicht anders angegeben bei Normalbedingungen (20 °C und 1013 mbar) durchgeführt worden. Werden nachfolgend Mittelwerte angegeben, so handelt es sich, wenn nicht anderes angegeben, um Gewichtsmittel.

Das Wortfragment "Poly" umfasst im Zusammenhang mit dieser Erfindung nicht nur Verbindungen mit zumindest 3 Wiederholungseinheiten eines oder mehrerer Monomere im Molekül, sondern insbesondere auch solche Zusammensetzungen von Verbindungen, die eine Molekulargewichtsverteilung aufweisen und dabei ein mittleres Molekulargewicht von mindestens 200 g/mol besitzen. Bei dieser Definition ist dem Umstand Rechnung getragen, dass es auf dem betrachteten Gebiet der Technik üblich ist, solche Verbindungen bereits als Polymere zu bezeichnen, auch wenn sie nicht einer Polymerdefinition analog OECD- oder REACH-Richtlinien zu genügen scheinen.

Im erfindungsgemäßen Verfahren werden SiH-funktionelle Siloxane eingesetzt. Diese sind dem Fachmann als solche bekannt. Vorzugsweise erfolgt die Bereitstellung der SiH-funktionellen Siloxane für das erfindungsgemäße Verfahren durch die Durchführung des aus dem Stand der Technik bekannten Verfahrens der Äquilibrierung, bevorzugt an sulfonsaurem Harz. Die Äquilibrierung der verzweigten oder linearen, ggf. hydrosilylierten, Poly(organo)siloxane mit end- und/oder seitenständigen SiH-Funktionen wird im Stand der Technik, z. B. in den Schriften EP 1 439 200 A1, DE 10 2007 055 485 A1 und DE 10 2008 041 601 ausführlich beschrieben. Diese Schriften werden hiermit als Referenz eingeführt und gelten als Teil der Offenbarung der vorliegenden Erfindung.

Als Reaktionspartner der SiH-funktionellen Siloxane im Sinne der Hydrosilylierung können beliebige organische ungesättigte Verbindungen eingesetzt werden. Vorzugsweise werden terminal ungesättigte organische Verbindungen eingesetzt.

So können neben terminal ungesättigten Allyl-funktionellen Polyethern z.B. auch andere niedermolekulare terminal ungesättigte organische Verbindungen eingesetzt werden.

Bevorzugt können terminal-ungesättigte Polyether wie Allyl- oder Methallyl-funktionelle Polyether eingesetzt werden, besonders bevorzugt Allylpolyether. Diese Polyether können nach den bekannten Verfahren, die dem Stand der Technik zu entnehmen sind, hergestellt werden. Die Alkoxylierung von ungesättigten Startverbindungen kann sowohl unter Basen-, Säuren- oder Doppelmetallcyanid-(DMC)-Katalyse hergestellt werden.

Als Einstieg in diese Thematik sei auf die Monografie *"Alkylene oxides and their polymers"* von F.E. Bailey, Marcel Dekker Verlag, 1991 verwiesen. Die Herstellung und Verwendung von DMC-Alkoxylierungskatalysatoren ist seit den 1960er Jahren bekannt und werden zum Beispiel in US 3,427,256, US 3,427,334, US 3,427,335, US 3,278,457, US 3,278,458 oder US 3,278,459 dargestellt. Noch wirksamere DMC-Katalysatoren, im speziellen Zink-Cobalt-Hexacyanokomplexe wurden in der Folgezeit entwickelt, z.B. in US 5,470,813 und US 5,482,908.

Die terminalen Hydroxygruppen der Polyether können frei bleiben oder können teilweise oder komplett modifiziert werden, um die optimale Kompatibilität in der späteren Anwendungsmatrix einstellen zu können.

Als Modifikation sind Umesterungen, Veresterungen oder Veretherungen ebenso denkbar wie weitere Kondensations- oder Additionsreaktionen mit z. B. Isocyanaten. Die terminalen Hydroxylgruppen der Polyether im Rahmen der vorliegenden Erfindung bleiben bevorzugt frei oder liegen in acetylierter oder methylierter Form vor.

Als terminal ungesättigte organische Verbindungen können bevorzugt Alken-Verbindungen, die noch weitere Substituenten tragen, eingesetzt werden. Es können zum Beispiel Allylglykol, Allylglycidether, Glycerinmonoallylether, Allylanisol, Allylphenol, Eugenol, Hexenol, C6-C20-Alken, Vinylcyclohexenmonooxid sowie Undecylensäure oder Undecylensäuremethylester eingesetzt werden, insbesondere bevorzugt Allylglykol, Tetradecen, Hexadecen, Octadecen, Eugenol und Glycerinmonoallylether.

Neben oder anstelle von terminal ungesättigten Verbindungen können auch Verbindungen mit internen Doppelbindungen wie beispielsweise Norbornen-Derivate oder auch interne Alkin-Verbindungen eingesetzt werden. Besonders bevorzugt werden allerdings terminal ungesättigte Alkene und Polyether eingesetzt.

Wie in der Einleitung bereits erwähnt können im erfindungsgemäßen Verfahren jegliche Edelmetallkatalysatoren verwendet werden, die eine SiC-Verknüpfungsreaktion zwischen einem SiH-funktionellen Polysiloxan und einer ungesättigten Verbindung katalysieren. Solche sind dem Fachmann wohlbekannt.

Einsetzbare katalytisch aktive Edelmetall-Verbindungen basieren insbesondere auf Komplexen des Platins, Palladiums, Rhodiums, Rutheniums, Iridiums und Osmiums.

Bevorzugt einsetzbar sind im Rahmen der vorliegenden Erfindung Platinverbindungen wie Hexachloroplatinsäure, cis-Diamminoplatin^{(II)}chlorid und Karstedt-Katalysator.

Pt⁽⁰⁾-Verbindungen wie der Karstedt-Komplex sind erfindungsgemäß als Katalysatoren besonders bevorzugt einsetzbar, insbesondere bevorzugt sind Komplexe mit Divinyl-tetramethyldisiloxanresten.

Darüber hinaus eignen sich aber auch andere stabile nullwertige Platin-Olefinkomplexe wie z.B. Bis-1,5-cyclooctadien-platin⁽⁰⁾ und Tris-norbornen-platin⁽⁰⁾, Di-platin-tris(heptadien-1,6), Platin-(η2,η2-1,2,6,7-heptadien-1,6)(η2-1,2-heptadien-1,6) und Platin-(η2-ethylen)( η2,η2-1,2,6,7-heptadien-1,6).

Als Adsorbentien lassen sich insbesondere beliebige anorganische Salze mit bevorzugt großer Oberfläche einsetzen. Es eignen sich saure oder basische Ionenaustauscher, Chelatisierungsagenzien oder funktionalisierte Silicagele ebenso wie aktivierter Kohlenstoff oder Carbon black.

Es entspricht einer bevorzugten Ausführungsform der Erfindung, wenn als Adsorbentien Aktivkohlen, Aluminiumoxide, Magnesiumsilikate, Aluminiumsilikate, wie vorzugsweise Zeolithe oder Kaolinit, Kieselgele, funktionalisierte Silicagele, Tonerden, Ruße, faserförmige oder mikrokristalline Cellulose, synthetische poröse Adsorberharze, Polymeradsorbentien, wie z.B. vernetzte Styrolpolymere, Molekularsieb, saure oder basische Ionenaustauscher, und/oder Chelatisierungsagenzien eingesetzt werden, wobei Aluminiumsilikate und/oder Magnesiumsilikate besonders bevorzugt sind.

Bevorzugt einsetzbar sind relativ preisgünstig zu erwerbende Aluminiumsilikate oder Magnesiumsilikate, die vorteilhafterweise mit großer Oberfläche ausgestattet sind.

Die Firma *The Dallas Group of America Inc.* vertreibt unter dem Namen Magnesol^{®} ein breites Produktspektrum an Magnesiumsilikaten, die bevorzugt zur Aufreinigung von Biodiesel Einsatz finden.

Das Produktspektrum offeriert sowohl unterschiedliche Molverhälnisse von Magnesiumoxid zu Siliciumdioxid, als auch diverse Partikelgrößen der Adsorbentien sowie unterschiedlich großer Oberfläche des Feststoffs, um ein optimales Wirkprofil für die jeweilige Anwendung anbieten zu können.

Im Sinne der vorliegenden Erfindungsmeldung besonders vorteilhaft einsetzbar sind Verbindungen mit einem Molverhältnis von MgO : SiO₂ von (1 : 5) bis (1 : 1), bevorzugt (1 : 3,6) bis (1 : 2,7), besonders bevorzugt (1 : 3,0) und (1 : 2,7).

Die spezifische Oberfläche (BET) des Feststoffs kann bevorzugt mindestens 50 bis 700 m²/g, besonders bevorzugt mindestens 70 m²/g und in einer weiteren Ausführungsform bevorzugt mindestens 350 m²/g betragen, bestimmbar mittels BET-Verfahren, insbesondere bestimmbar gemäß DIN ISO 9277:2014-01.

Die mittlere Partikelgröße kann vorzugsweise 10 bis 100 µm, bevorzugt 20 bis 80 µm betragen. Insbesondere vorteilhaft einsetzbar im erfindungsgemäßen Verfahren sind Magnesiumsilikate mit einer mittleren Partikelgröße zwischen 40 bis 60 µm. Die mittlere Partikelgröße im Sinne dieser Erfindung ist der D50 Wert, angegeben als volumetrischer Durchmesser. D50 bedeutet, dass 50% der Partikel kleiner sind als der angegebene Wert. Der D50 Wert kann insbesondere per Laserdiffraktometrie bestimmt werden.

Als weitere Firma, die geeignete Adsorbentien vertreibt, sei beispielsweise *Kyowa Chemical Industry Co., Ltd.* genannt, die unter dem Namen KYOWAAD^{®} ein breites Produktspektrum anbietet. Neben dem Magnesiumsilikat KYOWAAD^{®} 600 beispielsweise auch ein Aluminiumsilikat KYOWAAD^{®} 700 sowie ein Hydrotalcit KYOWAAD^{®} 500.

Weitere vorteilhaft einsetzbare Aluminiumsilikate sind das natürlich vorkommenden Kaolin (CAS 1332-58-7), auch Tonerde oder China Clay genannt oder insbesondere Kaolinit (CAS 1318-74-7).

Überraschenderweise wurde gefunden, dass der erfindungsgemäße Einsatz der Adsorbentien, die als eine weitere, separate Komponente zugegeben werden, im Rahmen der Edelmetall-katalysierten Hydrosilylierung eines H-funktionellen Siloxans mit einer ungesättigten organischen Verbindung zu farbreduzierten, vorzugsweise farblosen Finalprodukten führt.

Die Zugabe der Adsorbentien kann vor und/oder während der Edelmetall-katalysierten Hydrosilylierung erfolgen.

In einer bevorzugten Ausführungsform des erfindungsgemäßen Verfahrens werden ein SiH-Siloxan und eine ungesättigte organische Verbindung zusammen mit den Adsorbentien, insbesondere umfassend Magnesiumsilikat, vorgelegt und temperiert; dann gibt man den Edelmetallkatalysator zu und lässt bei der, dem Reaktionsgemisch angepassten optimalen Temperatur rühren, bis der SiH-Wert des Reaktionsgemisches einen nahezu quantitativen Umsatz (> 99%) belegt.

In einer alternativen Ausführungsform kann es jedoch auch vorteilhaft sein, zunächst die ungesättigte organische Verbindung mit Adsorbentien und Katalysator vorzulegen und zu temperieren und erst dann das SiH-funktionelle Siloxan kontrolliert zuzudosieren.

In einer weiteren bevorzugten Ausführungsform kann es vorteilhaft sein, erst das SiH-funktionelle Siloxan gemeinsam mit dem Adsorbens vorzulegen, den Katalysator zuzusetzen und erst dann die ungesättigte organische Verbindung kontrolliert zuzudosieren.

Nach beendeter Hydrosilylierung kann das Reaktionsgemisch bei Bedarf destillativ aufgereinigt werden, um beispielsweise niedermolekulare Verunreinigungen zu entfernen.

Abschließend wird das Reaktionsgemisch insbesondere per Filtration von festen Bestandteilen wie beispielsweise Adsorbens befreit und man erhält ein farbreduziertes, insbesondere farbloses Hydrosilylierungsprodukt, insbesondere Polyethersiloxan.

Überraschenderweise wurde weiterhin gefunden, dass in einer besonders bevorzugten Ausführungsform des Verfahrens, umfassend den Einsatz von Adsorbens und Wasser, sogar ein qualitativ noch besseres Produkt erhalten werden kann.

So kann die optische Erscheinung (insbesondere in Form eines noch helleren Produktes) noch weiter verbessert werden, insbesondere kann der dafür ursächliche Pt-Gehalt noch weiter reduziert werden, wobei die Anwesenheit von Wasser und Absorbens keinerlei negativen Einfluss auf die Reaktion hat.

Die Menge der eingesetzten Adsorbentien kann in breiten Bereichen gewählt werden. Insbesondere hinsichtlich Kosten-Nutzen-Betrachtung haben sich Mengen von vorzugsweise 0,05 bis 5 Gew.-% Adsorbens im Verhältnis zur Gesamtansatzmenge als sinnvoll erwiesen. Besonders bevorzugt werden 0,1 bis 2 Gew.-% und insbesondere bevorzugt 0,2 bis 1 Gew.-% Adsorbens eingesetzt, Gew.-% bezogen auf die gesamte Reaktionsmasse. Dies entspricht einer besonders bevorzugten Ausführungsform der Erfindung.

Die optional eingesetzte Wassermenge kann in breiten Bereichen gewählt werden. Insbesondere hinsichtlich Kosten-Nutzen-Betrachtung haben sich Mengen von vorzugsweise 0,05 bis 50 Gew.-% Wasser im Verhältnis zur Gesamtansatzmenge als sinnvoll erwiesen. Besonders bevorzugt werden 0,5 bis 5 Gew.-% und insbesondere bevorzugt 1 bis 3 Gew-% Wasser eingesetzt, Gew.-% bezogen auf die gesamte Reaktionsmasse. Dies entspricht einer besonders bevorzugten Ausführungsform der Erfindung.

Das erfindungsgemäße Verfahren wird bevorzugt unter inerter Atmosphäre, bevorzugt unter Argon- oder Stickstoffstrom und bei Temperaturen von vorzugsweise 50 bis 130°C durchgeführt.

Mittels des erfindungsgemäßen Verfahrens können z. B. die nachfolgend beschriebenen Polysiloxanverbindungen hergestellt werden:
Bevorzugte erfindungsgemäß erhältliche Polysiloxanverbindungen sind solche der Formel (I)

Mₐ M'_{b} M"_{c} D_{d} D'ₑ D"_{f} T_{g} Qₕ Formel (I)

und zeichnen sich dadurch aus, dass
- M: = [R¹₃SiO_{1/2}]
- M': = [R² R¹₂SiO_{1/2}]
- M": = [R³ R¹₂SiO_{1/2}]
- D: = [R¹₂SiO_{2/2}]
- D': = [R² R¹SiO_{2/2}]
- D": = [R³ R¹SiO_{2/2}]
- T: = [R¹SiO_{3/2}]
- Q: = [SiO_{4/2}]

- a: = 0 - 20, bevorzugt 0 - 10, insbesondere bevorzugt 2,
- b: = 0 - 20, bevorzugt 0 - 10, insbesondere bevorzugt 0 oder 2,
- c: = 0 - 20, bevorzugt 0 - 10, insbesondere bevorzugt 0 oder 2,
- d: = 0 - 1000, bevorzugt 0 - 500, insbesondere bevorzugt 0 - 200,
- e: = 0 - 30, bevorzugt 1 - 15, insbesondere bevorzugt 1 - 10,
- f: = 0 - 30, bevorzugt 0 - 15, insbesondere bevorzugt 0 - 10,
- g: = 0 - 20, bevorzugt 0 - 10, insbesondere bevorzugt 0 - 5,
- h: = 0 - 20, bevorzugt 0 - 15, insbesondere bevorzugt 0 - 5,
mit der Maßgabe, dass die Summe aus a + b + c + d + e + f + g + h ≥ 3, sowie die Summe aus b + c + e + f ≥ 1 sein muss, sowie die Summe aus e + f bevorzugt ≥ 1
und
- R¹ =: unabhängig voneinander gleiche oder verschiedene Kohlenwasserstoffreste mit 1 - 7 Kohlenstoffatomen oder H, bevorzugt Methyl, Ethyl oder Phenyl, insbesondere bevorzugt Methyl,
- R² =: unabhängig voneinander gleiche oder verschiedene Polyetherreste,
- R³ =: unabhängig voneinander gleiche oder verschiedene Kohlenwasserstoffreste mit 8 - 20 Kohlenstoffatomen, die auch Heteroatome enthalten und weiter substituiert sein können, bevorzugt handelt es sich um SiC-verknüpfte Reste resultierend aus Alkindiol, sowie deren Alkoxylate, Allylglykol, Allyloxyethanol, Allylglycidether, Glycerinmonoallylether, Allylanisol, Eugenol, Hexenol, Hexadecen, Octadecen, Undecylensäure und Undecylensäuremethylester, insbesondere bevorzugt Hexadecen, Octadecen, Eugenol und Glycerinmonoallylether.

Die erfindungsgemäß bevorzugt resultierenden Polysiloxanverbindungen der Formel (I) sind vorzugsweise erhältlich durch das oben beschriebene erfindungsgemäße Verfahren.

Die erfindungsgemäß resultierenden Hydrosilylierungsprodukte, vorzugsweise die bevorzugten Polysiloxanverbindungen, insbesondere der Formel (I), können für vielfältige Anwendungen eingesetzt werden, insbesondere ist der Einsatz als grenzflächenaktive Substanzen zu nennen. Insbesondere in Betracht kommt die Verwendung als Dispergieradditiv, Entschäumer, Benetzungshilfsmittel, Hydrophobierungsmittel oder vernetzendes Additiv vorzugsweise zum Einsatz in Pasten, Farben, Lacken, Überzügen, Beschichtungen, und/oder Anstrichmitteln, sowie in Antitranspirantien/Deodorantien, sowie in pharmazeutischen Formulierungen. Hinzu kommt außerdem die Verwendung in Reinigungs- und/oder Pflegeformulierungen geeignet zur Reinigung und/oder Pflege harter Oberflächen und/oder geeignet zur Reinigung, Behandlung und Nachbehandlung von Textilien, sowie in kosmetischen Produkten. Hinzu kommt weiterhin die Verwendung als Schaumstabilisatoren oder Schaumadditive für Polyurethanschäume. Hinzu kommt die Verwendung als Adjuvant zur Verbesserung der Wirkung von Pflanzenschutzwirkstoffen und/oder als Träger für Pflanzenschutzwirkstoffe, wobei die Pflanzenschutzwirkstoffe vorzugsweise aus mikrobiologischen Pflanzenschutzwirkstoffen ausgewählt sind.

### Messmethoden:

Zur Bestimmung von Parametern oder Messwerten werden im Rahmen der vorliegenden Erfindung vorzugsweise die nachfolgend beschriebenen Methoden verwendet. Insbesondere wurden diese Methoden in den Beispielen des vorliegenden Schutzrechts verwendet.

Die Bestimmung des SiH-Umsatzes der Hydrosilylierung erfolgt durch Butylat-katalysierte Freisetzung des in der Probe enthaltenen (Rest-)Si-H als elementarem Wasserstoff und dessen quantitativer Bestimmung.

Die Pt-Gehaltsbestimmung erfolgt mittels matrixangepasster Kalibrierlösungen an der ICP-OES (Inductively Coupled Plasma-Optical Emission Spectrometry). Hierzu wird die zu analysierende Probe zunächst genau eingewogen und in einem Mikrowellen-Aufschluss mit HNO₃/HF aufgeschlossen. Je nach Si-Gehalt müssen - wie dem Fachmann bekannt - unterschiedliche Mengen an HF benutzt werden. Anschließend wird die Säure abgedampft und mit Königswasser aufgenommen und auf ein definiertes Volumen aufgefüllt.

Die Gehaltsbestimmung erfolgt dann durch Einspritzen in das ICP-OES. Die Probe mit unbekannter Zusammensetzung wird direkt im Anschluss an die Kalibration gemessen. Nach der Messung wird mit einer Kalibrationslösung überprüft, ob bei der Messung stabile Messbedingungen vorlagen. Die Werte wurden in einer Doppelbestimmung bestimmt und die Angabe des Ergebnisses erfolgt als Mittelwert beider Messungen in ppm; bis 2 ppm, mit einer Stelle nach dem Komma genau.

Die nasschemische Analytik wurde in Anlehnung an internationale Standardmethoden durchgeführt: lodzahl (IZ; DGF C-V 11 a (53); Säurezahl (SZ; DGF C-V 2); OH-Zahl (ASTM D 4274 C).

Die Hazen-Farbzahl wurde bestimmt nach DIN EN ISO 6271 (2005): Bestimmung der Farbe klarer Flüssigkeiten nach der Platin-Cobalt-Skala.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Als Karstedt-Katalysator wurde in den nachfolgend aufgeführten Beispielen Platinum(0)-1,3-divinyl-1,1,3,3-tetramethyldisiloxan 2%ig gelöst in Xylol eingesetzt.

MAGNESOL^{®} Polysorb 3040 ist ein Magnesiumsilikat der Firma "The Dallas Group of America Inc." Es wurde in einigen Beispielen eingesetzt, siehe nachfolgende Beispiele.

*KYOWAAD*^{®} *600* ist ein Magnesiumsilikat der Firma *Kyowa Chemical Industriy Co., Ltd.* Es wurde in einigen Beispielen eingesetzt, siehe nachfolgende Beispiele.

Kaolinite, natural (CAS: 1318-74-7) ist ein Aluminiumsilikat, bezogen von Sigma-Aldrich. Es wurde in einem Beispiel eingesetzt, siehe nachfolgende Beispiele.

### Beispiele:

### Beispiel 1a: Synthese eines HMTS-basierten Polyethersiloxans (Vergleichsbeispiel):

In einem mit Tropftrichter mit Druckausgleichsrohr, Thermometer, Intensivkühler und Sigma-Rührer versehenen 1 L Planschliffkolben werden 226,6 g eines Allylpolyethers (Ethoxylat von Allylalkohol mit einer IZ von 63 g lod/100 g) vorgelegt und unter Rühren und Argonüberleitung auf 90°C erwärmt. Dann wird Karstedt-Katalysator mit einer Micropipette zugegeben (c (Ansatz) = 4 ppm Pt). Anschließend wird über den Tropftrichter das Heptamethyltrisiloxan mit einer Masse von 100 g (HMTS mit SiH = 4,50 mol/kg) in ca. 30 Minuten dergestalt zugetropft, dass die Temperatur des Reaktionsgemisches nicht mehr als 115°C erreicht. Nach beendeter Zugabe wird noch 1 h bei 110°C nachgerührt und dann der SiH-Umsatz bestimmt. Es ergab sich der in Tabelle 1 angegebene SiH-Umsatz bestimmt nach der Natriumbutylatmethode. Anschließend wird 1 h bei 120°C und p < 10 mbar destilliert, um leicht flüchtige Produktbestandteile zu entfernen und abschließend über einen Schichtenfilter filtriert.

### Beispiel 1b: Synthese eines HMTS-basierten Polyethersiloxans (Vergleichsbeispiel):

Das Beispiel wurde analog Beispiel 1a durchgeführt, mit dem Unterschied, dass nach Zugabe des Allylpolyethers auch die in Tabelle 1 angegebene Menge Wasser zugegeben wurden.

### Beispiel 1c: Synthese eines HMTS-basierten Polyethersiloxans (erfindungsgemäß):

Das Beispiel wurde analog Beispiel 1a durchgeführt, mit dem Unterschied, dass nach Zugabe des Allylpolyethers die in Tabelle 1 angegebene Menge *MAGNESOL*^{®} *Polysorb 3040* (bezogen auf Gesamtansatz) zugegeben wurde.

### Beispiel 1d: Synthese eines HMTS-basierten Polyethersiloxans (erfindungsgemäß):

Das Beispiel wurde analog Beispiel 1a durchgeführt, mit dem Unterschied, dass nach Zugabe des Allylpolyethers auch die in Tabelle 1 angegebene Mengen *MAGNESOL*^{®} *Polysorb 3040* und Wasser (jeweils bezogen auf Gesamtansatz) zugegeben wurden.

### Beispiel 1e: Synthese eines HMTS-basierten Polyethersiloxans (erfindungsgemäß):

Das Beispiel wurde analog Beispiel 1a durchgeführt, mit dem Unterschied, dass nach Zugabe des Allylpolyethers auch die in Tabelle 1 angegebene Mengen *MAGNESOL*^{®} *Polysorb 3040* und Wasser (jeweils bezogen auf Gesamtansatz) zugegeben wurden.

### Beispiel 1f: Synthese eines HMTS-basierten Polyethersiloxans (erfindungsgemäß):

Das Beispiel wurde analog Beispiel 1a durchgeführt, mit dem Unterschied, dass nach Zugabe des Allylpolyethers auch die in Tabelle 1 angegebene Mengen Kaolinit und Wasser (jeweils bezogen auf Gesamtansatz) zugegeben wurden.

**Tabelle 1: Additivierungen und analytische Daten der Beispiele 1a-f**

| **Beispiel** | **SiH-Umsatz** | **Adsorbent** | **Wasser** | **Hazen** | **Pt Gehalt** |
|---|---|---|---|---|---|
| | **[%]** | **[%]** | **[%]** | | **[ppm]** |
| 1a | 99,2 | 0 | 0 | 80 | 4,0 |
| 1b | 99,9 | 0 | 0,5 | 80 | 3,0 |
| 1c | 99,8 | 0,5 | 0 | 48 | 2,0 |
| 1d | 99,8 | 0,5 | 0,5 | 30 | 1,6 |
| 1e | 99,9 | 0,5 | 1,0 | 11 | 0,7 |
| 1f | 98,9 | 0,125 | 0,5 | 7 | 0,2 |

### Beispiel 2a: Synthese eines C16-Alpha-Olefin-basierten Polyalkylsiloxans (Vergleichsbeispiel):

In einem mit Tropftrichter mit Druckausgleichsrohr, Thermometer und Sigma-Rührer versehenen Planschliffkolben werden 250 g eines kammständigen SiH-Siloxans

(SiH = 6,88 mol/kg, M₂D_{5,4}D^{H}_{6,6}) vorgelegt und unter Rühren und Argonüberleitung auf 90°C erwärmt. Dann wird Karstedt-Katalysator (c (Ansatz) = 3 ppm Pt) mit einer Micropipette zugegeben. Anschließend wird über den Tropftrichter das C16 Alpha-Olefin mit der Masse 443,9 g in ca. 40 Minuten dergestalt zugetropft, dass die Temperatur des Reaktionsgemisches nicht mehr als 115°C erreicht. Nach beendeter Zugabe wird noch 1 h bei 110°C nachgerührt und dann der SiH-Umsatz bestimmt. Es ergab sich der in Tabelle 2 angegebene SiH-Umsatz bestimmt nach der Natriumbutylatmethode.

### Beispiel 2b: Synthese eines C16-Alpha-Olefin-basierten Polyalkylsiloxans (erfindungsgemäß):

Das Beispiel wurde analog Beispiel 2a durchgeführt, mit dem Unterschied, dass nach Zugabe des Siloxans auch die in Tabelle 2 angegebene Mengen *MAGNESOL*^{®} *Polysorb 3040* und Wasser (jeweils bezogen auf Gesamtansatz) zugegeben wurden.

### Beispiel 2c: Synthese eines C16-Alpha-Olefin-basierten Polyalkylsiloxans (erfindungsgemäß):

Das Beispiel wurde analog Beispiel 2b durchgeführt, mit dem Unterschied, dass das C16 Alpha-Olefin vorgelegt und das Siloxan zudosiert wurde.

**Tabelle 2: Additivierungen und analytische Daten der Beispiele 2a-c**

| **Beispiel** | **SiH-Umsatz** | **Adsorbent** | **Wasser** | **Hazen** | **Pt Gehalt** |
|---|---|---|---|---|---|
| | **[%]** | **[%]** | **[%]** | | **[ppm]** |
| 2a | 97,9 | 0 | 0 | **38** | **2,0** |
| 2b | 97,5 | 0,25 | 0,5 | **21** | **0,9** |
| 2c | 99,9 | 0,25 | 0,5 | **11** | **0,6** |

### Beispiel 3a: Synthese eines kammständigen Polyethersiloxans (Vergleichsbeispiel):

In einem mit Tropftrichter mit Druckausgleichsrohr, Thermometer, Intensivkühler und Sigma-Rührer versehenen 1 L Planschliffkolben werden 202,3 g eines methylierten Allylpolyethers (Ethoxylat von Allylalkohol mit einer IZ von 63,5 g lod/100 g dessen terminale OH-Gruppe methyliert war) vorgelegt und unter Rühren und Argonüberleitung auf 90°C erwärmt. Dann wird Karstedt-Katalysator mit einer Micropipette zugegeben (c (Ansatz) = 4 ppm Pt). Anschließend wird über den Tropftrichter das kammständige SiH-Siloxan mit der Masse 250 g (SiH = 1,50 mol/kg, M₂D₆D^{H}₁) in ca. 30 Minuten dergestalt zugetropft, dass die Temperatur des Reaktionsgemisches nicht mehr als 115°C erreicht. Nach beendeter Zugabe wird noch 5 h bei 110°C nachgerührt und dann der SiH-Umsatz bestimmt. Es ergab sich der in Tabelle 3 angegebene SiH-Umsatz bestimmt nach der Natriumbutylatmethode. Anschließend wird 2 h bei 120°C und p < 10 mbar destilliert, um leicht flüchtige Produktbestandteile zu entfernen und abschließend über einen Schichtenfilter filtriert.

### Beispiel 3b: Synthese eines kammständigen Polyethersiloxans (Vergleichsbeispiel):

Das Beispiel wurde analog Beispiel 3a durchgeführt, mit dem Unterschied, dass nach Zugabe des Allylpolyethers auch die in Tabelle 3 angegebene Menge Wasser zugegeben wurden.

### Beispiel 3c: Synthese eines kammständigen Polyethersiloxans (erfindungsgemäß):

Das Beispiel wurde analog Beispiel 3a durchgeführt, mit dem Unterschied, dass nach Zugabe des Allylpolyethers auch die in Tabelle 3 angegebene Menge *MAGNESOL*^{®} *Polysorb 3040* (bezogen auf Gesamtansatz) zugegeben wurden.

### Beispiel 3d: Synthese eines kammständigen Polyethersiloxans (erfindungsgemäß):

Das Beispiel wurde analog Beispiel 3a durchgeführt, mit dem Unterschied, dass nach Zugabe des Allylpolyethers auch die in Tabelle 3 angegebene Mengen *MAGNESOL^{®} Polysorb 3040* und Wasser (jeweils bezogen auf Gesamtansatz) zugegeben wurden.

### Beispiel 3e: Synthese eines kammständigen Polyethersiloxans (erfindungsgemäß):

Das Beispiel wurde analog Beispiel 3d durchgeführt, mit dem Unterschied, dass als Absorbent *KYOWAAD*^{®} *600* statt *MAGNESOL*^{®} *Polysorb 3040* zugegeben wurden.

**Tabelle 3: Additivierungen und analytische Daten der Beispiele 3a-e**

| **Beispiel** | **SiH-Umsatz** | **Adsorbent** | **Wasser** | **Hazen** | **Pt Gehalt** |
|---|---|---|---|---|---|
| | **[%]** | **[%]** | **[%]** | | **[ppm]** |
| 3a | >99% | 0 | 0 | **22** | **1,1** |
| 3b | >99% | 0 | 0,5 | **36** | **1,5** |
| 3c | 98,1% | 0,25 | 0 | **5** | **0,5** |
| 3d | >99% | 0,25 | 0,5 | **4** | **0,2** |
| 3e | >99% | 0,25 | 0,5 | **1** | **0,2** |

### Beispiel 4a: Synthese eines linearen Polyethersiloxans (Vergleichsbeispiel):

In einem mit Tropftrichter mit Druckausgleichsrohr, Thermometer, Intensivkühler und Sigma-Rührer versehenen 1 L Planschliffkolben werden nacheinander 381,8 g eines Allylpolyethers (Copolymerisat von EO (60%) und PO (40%) an Allylalkohol mit einer IZ von 49 g Iod/100 g) sowie 300 g Siloxan (SiH-Wert = 1,82 mol/kg, M₂*H*D₁₃) vorgelegt und unter Rühren und Argonüberleitung auf 55°C erwärmt. Dann wird Karstedt-Katalysator mit einer Micropipette zugegeben (c (Ansatz) = 6 ppm Pt). Es wird auf 90°C aufgeheizt und gegebenenfalls gegengekühlt, damit eine Temperatur von 110°C nicht überschritten wird. Anschließend wird noch 1 h bei 110°C nachgerührt und dann der SiH-Umsatz bestimmt. Es ergab sich der in Tabelle 4 angegebene SiH-Umsatz bestimmt nach der Natriumbutylatmethode. Anschließend wird 1 h bei 120°C und p < 10 mbar destilliert, um leicht flüchtige Produktbestandteile zu entfernen und abschließend über einen Schichtenfilter filtriert.

### Beispiel 4b: Synthese eines linearen Polyethersiloxans (erfindungsgemäß):

Das Beispiel wurde analog Beispiel 4a durchgeführt, mit dem Unterschied, dass nach Zugabe des Allylpolyethers und des Siloxans auch die in Tabelle 4 angegebene Mengen *MAGNESOL*^{®} *Polysorb 3040* und Wasser (jeweils bezogen auf Gesamtansatz) zugegeben wurden.

**Tabelle 4: Additivierungen und analytische Daten der Beispiele 4a-b**

| **Beispiel** | **SiH-Umsatz** | **Adsorbent** | **Wasser** | **Hazen** | **Pt Gehalt** |
|---|---|---|---|---|---|
| | **[%]** | **[%]** | **[%]** | | **[ppm]** |
| 4a | 99,9 | 0 | 0 | **91** | **4,0** |
| 4b | 99,9 | 0,5 | 1 | **36** | **1,7** |

### Fazit:

Bei Betrachtung der in den Tabellen 1-4 angegebenen Hazen-Farbzahlen und Pt-Gehalten ist dem Fachmann ersichtlich, dass die besten Produkte mit den geringsten Hazen-Farbzahlen und niedrigsten Pt-Gehalten durch das erfindungsgemäße Verfahren erhalten wurden.

Die Durchführung des Verfahrens in Anwesenheit erfindungsgemäßer Adsorbentien, insbesondere von Magnesiumsilikat, führt bereits zu qualitativ viel hochwertigeren Produkten. Diese Qualität wird durch den Einsatz der Kombination Wasser und Adsorbentien, insbesondere Magnesiumsilikat, jedoch noch weiter erhöht.

Die Anwesenheit des Wassers hat keinerlei negativen Einfluss auf die Hydrosilylierungsreaktion.

## Patentansprüche

1. Verfahren zur Herstellung von organisch modifizierten Polysiloxanen und/oder Silanen durch Hydrosilylierung, umfassend folgende Schritte:
a) Umsetzung eines SiH-funktionellen Siloxans und/oder Silans mit einer ungesättigten organischen Verbindung in Anwesenheit eines Edelmetallkatalysators sowie optional in Anwesenheit von Wasser,
b) optionale Destillation,
c) abschließende Feststoffabtrennung, insbesondere durch Filtration, **dadurch gekennzeichnet, dass** in dem Schritt a) Adsorbentien zum Einsatz gelangen, die als eine weitere, separate Komponente zugegeben werden.

2. Verfahren gemäß Anspruch 1, **dadurch gekennzeichnet, dass** die ungesättigten organischen Verbindungen terminal ungesättigte organischen Verbindungen umfassen, bevorzugt entsprechend terminal ungesättigte Alkenverbindungen sind, die ggf. noch weitere Substituenten tragen können, bevorzugt umfassend Allylglycidether, Glycerinmonoallylether, Allylglykol, Allyloxyethanol, Allylanisol, Allylphenol, Eugenol, Hexenol, C6-C20-Alken, Vinylcyclohexenmonooxid darunter bevorzugt Allylglykol, Hexadecen, Octadecen, sowie Undecylensäuremethylester, wobei insbesondere terminal-ungesättigte Polyether, wie Allyl- oder Methallyl-funktionelle Polyether eingesetzt werden, und ganz besonders bevorzugt terminal-ungesättigte Allylpolyether eingesetzt werden.

3. Verfahren gemäß Anspruch 1 oder 2, **dadurch gekennzeichnet, dass** die ungesättigten organischen Verbindungen solche mit internen Doppelbindungen wie beispielsweise Norbornen-Derivate oder auch interne Alkin-Verbindungen umfassen.

4. Verfahren gemäß zumindest einem der Ansprüche 1 bis 3, **dadurch gekennzeichnet, dass** als Edelmetallkatalysator Verbindungen oder Komplexe des Platins, Palladiums, Rhodiums, Rutheniums, Iridiums und/oder Osmiums eingesetzt werden, vorzugsweise Verbindungen oder Komplexen des Platins, insbesondere vom Karstedt-Typ.

5. Verfahren gemäß zumindest einem der Ansprüche 1 bis 4, **dadurch gekennzeichnet, dass** als Adsorbentien Aktivkohlen, Aluminiumoxide, Magnesiumsilikate, Aluminiumsilikate, wie vorzugsweise Zeolithe oder Kaolinit, Kieselgele, funktionalisierte Silicagele, Tonerden, Ruße, faserförmige oder mikrokristalline Cellulose, synthetische poröse Adsorberharze, Polymeradsorbentien, wie z.B. vernetzte Styrolpolymere, Molekularsieb, saure oder basische Ionenaustauscher, und/oder Chelatisierungsagenzien eingesetzt werden, wobei Aluminiumsilikate und/oder Magnesiumsilikate bevorzugt sind.

6. Verfahren gemäß zumindest einem der Ansprüche 1 bis 5, **dadurch gekennzeichnet, dass** das Adsorbens Magnesiumsilikate umfasst, vorzugsweise mit einem Molverhältnis von MgO : SiO₂ von (1 : 5) bis (1 : 1), bevorzugt (1 : 3,6) bis (1 : 2,7) , besonders bevorzugt (1 : 3,0) und (1 : 2,7).

7. Verfahren nach Anspruch 6, **dadurch gekennzeichnet, dass**
(a) die spezifische Oberfläche (BET) des Magnesiumsilikats vorzugsweise mindestens 50 bis 700 m²/g, besonders bevorzugt mindestens 70 m²/g, und/oder,
(b) die mittlere Partikelgröße 10 bis 100 µm, bevorzugt 20 bis 80µm, insbesondere 40 bis 60 µm beträgt.

8. Verfahren nach einem der Ansprüche 1 bis 7, **dadurch gekennzeichnet, dass** die Adsorbentien in einer Gesamtmenge von 0,05 bis 5 Gew-%, vorzugsweise 0,1 bis 2 Gew-% und insbesondere bevorzugt 0,2 bis 1 Gew-% eingesetzt werden, Gew.-% bezogen auf die gesamte Reaktionsmasse, wobei die Zugabe der Adsorbentien vor und/oder während der Edelmetall-katalysierten Hydrosilylierung erfolgt.

9. Verfahren nach einem der Ansprüche 1 bis 8, **dadurch gekennzeichnet, dass** die Hydrosilylierung unter inerter Atmosphäre, bevorzugt unter N₂-Atmosphäre oder ArgonAtmosphäre, und/oder, vorzugsweise und, bei Temperaturen von 50 bis 130°C durchgeführt wird.

10. Verfahren nach einem der Ansprüche 1 bis 9, **dadurch gekennzeichnet, dass** die Hydrosilylierung in Anwesenheit von Wasser durchgeführt wird, welches nach der Umsetzung destillativ entfernt wird, wobei die Menge an Wasser vorzugsweise 0,05 bis 50 Gew.-%, besonders bevorzugt 0,5 bis 5 Gew.-% und insbesondere bevorzugt 1 bis 3 Gew-% beträgt, Gew.-% bezogen auf die gesamte Reaktionsmasse.

## Claims

1. Process for producing organically modified polysiloxanes and/or silanes by hydrosilylation, comprising the following steps:
a) reaction of an SiH-functional siloxane and/or silane with an unsaturated organic compound in the presence of a noble metal catalyst and optionally in the presence of water,
b) optional distillation,
c) final removal of solids, especially by filtration, **characterized in that** adsorbents are used in step a), which are added as a further, separate component.

2. Process according to Claim 1, **characterized in that** the unsaturated organic compounds include terminally unsaturated organic compounds, preferably accordingly are terminally unsaturated alkene compounds, which may optionally bear further substituents, preferably comprising allyl glycidyl ether, glycerol monoallyl ether, allyl glycol, allyloxyethanol, allylanisole, allylphenol, eugenol, hexenol, C6-C20-alkene, vinylcyclohexene monoxide including preferably allyl glycol, hexadecene, octadecene, and methyl undecylenate, wherein use is especially made of terminally unsaturated polyethers, such as allyl- or methallyl-functional polyethers, and use is very particularly preferably made of terminally unsaturated allyl polyethers.

3. Process according to Claim 1 or 2, **characterized in that** the unsaturated organic compounds include those having internal double bonds such as norbornene derivatives or also internal alkyne compounds.

4. Process according to at least one of Claims 1 to 3, **characterized in that** the noble metal catalysts used are compounds or complexes of platinum, palladium, rhodium, ruthenium, iridium and/or osmium, preferably compounds or complexes of platinum, especially of the Karstedt type.

5. Process according to at least one of Claims 1 to 4, **characterized in that** the adsorbents used are activated carbons, aluminium oxides, magnesium silicates, aluminium silicates, such as preferably zeolites or kaolinite, silica gels, functionalized silica gels, clay earths, carbon blacks, fibrous or microcrystalline celluloses, synthetic porous adsorber resins, polymer adsorbents, such as crosslinked styrene polymers, molecular sieves, acidic or basic ion exchangers, and/or chelating agents, wherein aluminium silicates and/or magnesium silicates are preferred.

6. Process according to at least one of Claims 1 to 5, **characterized in that** the adsorbent comprises magnesium silicates, preferably at a molar ratio of MgO: SiO₂ of (1:5) to (1:1), preferably (1:3.6) to (1:2.7), particularly preferably (1:3.0) and (1:2.7).

7. Process according to Claim 6, **characterized in that** (a) the specific surface area (BET) of the magnesium silicate is preferably at least 50 to 700 m²/g, particularly preferably at least 70 m²/g, and/or (b) the average particle size is 10 to 100 µm, preferably 20 to 80 µm, especially 40 to 60 µm.

8. Process according to any of Claims 1 to 7, **characterized in that** the adsorbents are used in a total amount of 0.05% to 5% by weight, preferably 0.1% to 2% by weight and especially preferably 0.2% to 1% by weight, % by weight based on the total reaction mass, wherein the adsorbents are added before and/or during the noble metal-catalysed hydrosilylation.

9. Process according to any of Claims 1 to 8, **characterized in that** the hydrosilylation is carried out under an inert atmosphere, preferably under an N₂ atmosphere or argon atmosphere, and/or, preferably and, at temperatures of 50°C to 130°C.

10. Process according to any of Claims 1 to 9, **characterized in that** the hydrosilylation is carried out in the presence of water, which is removed by distillation after the reaction, wherein the amount of water is preferably 0.05% to 50% by weight, particularly preferably 0.5% to 5% by weight and especially preferably 1% to 3% by weight, % by weight based on the total reaction mass.

## Revendications

1. Procédé de préparation de polysiloxanes et/ou de silanes organiquement modifiés par hydrosilylation, comprenant les étapes suivantes :
a) transformation d'un siloxane et/ou d'un silane à fonctionnalité SiH avec un composé organique insaturé en présence d'un catalyseur de métal noble et éventuellement en présence d'eau,
b) distillation éventuelle,
c) séparation finale de solides, en particulier par filtration,
**caractérisé en ce que** l'on utilise dans l'étape a) des adsorbants ajoutés en tant qu'autre composant séparé.

2. Procédé selon la revendication 1, **caractérisé en ce que** les composés organiques insaturés comprennent des composés organiques insaturés en position terminale, de préférence des composés alcènes insaturés en position terminale correspondants, qui peuvent le cas échéant encore porter d'autres substituants, comprenant de préférence l'allylglycidyléther, le monoallyléther de glycérol, l'allylglycol, l'allyloxyéthanol, l'allylanisole, l'allylphénol, l'eugénol, l'hexénol, les alcènes en C6-C20, le monoxyde de vinylcyclohexène, parmi lesquels de préférence l'allylglycol, l'hexadécène, l'octadécène ainsi que l'ester méthylique de l'acide undécylénique ; les polyéthers insaturés en position terminale, tels que les polyéthers à fonctionnalité allyle ou méthyle, étant en particulier utilisés et les allylpolyéthers insaturés en position terminale étant utilisés de manière tout particulièrement préférée.

3. Procédé selon la revendication 1 ou 2, **caractérisé en ce que** les composés organiques insaturés comprennent ceux présentant des doubles liaisons internes tels que, par exemple, les dérivés de norbornène ou également les composés alcynes internes.

4. Procédé selon au moins l'une des revendications 1 à 3, **caractérisé en ce que** l'on utilise en tant que catalyseur de métal noble des composés ou complexes du platine, du palladium, du rhodium, du ruthénium, de l'iridium et/ou de l'osmium, de préférence des composés ou complexes du platine, en particulier du type Karstedt.

5. Procédé selon au moins l'une des revendications 1 à 4, **caractérisé en ce que** l'on utilise, en tant qu'adsorbants, le charbon actif, les oxydes d'aluminium, les silicates de magnésium, les silicates d'aluminium, tels que de préférence les zéolites ou la kaolinite, les gels de silice, les gels de silice fonctionnalisés, les argiles, les suies, la cellulose fibreuse ou microcristalline, les résines adsorbantes poreuses synthétiques, les adsorbants polymères tels que les polymères styréniques réticulés, les tamis moléculaires, les échangeurs ioniques acides ou basiques et/ou les agents chélateurs ; les silicates d'aluminium et/ou les silicates de magnésium étant préférés.

6. Procédé selon au moins l'une des revendications 1 à 5, **caractérisé en ce que** l'adsorbant comprend des silicates de magnésium, de préférence présentant un rapport molaire de MgO:SiO₂ de (1:5) à (1:1), de préférence de (1:3,6) à (1:2,7), de manière particulièrement préférée (1:3,0) et (1:2,7).

7. Procédé selon la revendication 6, **caractérisé en ce que**
(a) la surface spécifique (BET) du silicate de magnésium est de préférence d'au moins 50 à 700 m²/g, de manière particulièrement préférée d'au moins 70 m²/g et/ou
(b) la grosseur moyenne des particules est de 10 à 100 µm, de préférence de 20 à 80 µm, en particulier de 40 à 60 µm.

8. Procédé selon l'une des revendications 1 à 7, **caractérisé en ce que** les adsorbants sont utilisés en une quantité totale de 0,05 à 5% en poids, de préférence de 0,1 à 2% en poids et en particulier de préférence de 0,2 à 1% en poids, par rapport à la masse réactionnelle totale, l'ajout des adsorbants ayant lieu avant et/ou pendant l'hydrosilylation catalysée par un métal noble.

9. Procédé selon l'une des revendications 1 à 8, **caractérisé en ce que** l'hydrosilylation est réalisée sous atmosphère inerte, de préférence sous atmosphère de N₂ ou d'argon et/ou, de préférence et, à des températures de 50 à 130°C.

10. Procédé selon l'une des revendications 1 à 9, **caractérisé en ce que** l'hydrosilylation est réalisée en présence d'eau, qui est éliminée par distillation après la réaction, la quantité d'eau étant de préférence de 0,05 à 50% en poids, de manière particulièrement préférée de 0,5 à 5% en poids et en particulier de préférence de 1 à 3% en poids, par rapport à la masse réactionnelle totale.
